(19) Europäisches Patentamt
European Patent Office
Office européen des brevets

(11) Publication number: **0 038 046**
**B1**

(12) **EUROPEAN PATENT SPECIFICATION**

(45) Date of publication of patent specification: **29.02.84**

(21) Application number: **81102770.5**

(22) Date of filing: **10.04.81**

(51) Int. Cl.³: **C 07 C 103/46,**
C 07 C 103/48,
C 07 C 103/49,
C 07 C 149/243,
C 07 C 102/04,
A 61 K 31/095,
A 61 K 31/16, A 61 K 31/19

(54) **Pharmaceutical amides, and preparation, formulations and use thereof.**

(30) Priority: **11.04.80 GB 8011986**

(43) Date of publication of application:
**21.10.81 Bulletin 81/42**

(45) Publication of the grant of the patent:
**29.02.84 Bulletin 84/9**

(84) Designated Contracting States:
**BE CH DE FR GB LI LU NL SE**

(56) References cited:
**DE - B - 1 146 066**
**FR - A - 1 458 106**
**US - A - 2 463 939**

**Chemical Abstracts vol. 90, no. 21, 1979 Columbus, Ohio, USA J.P. SWERTS et al. "Is 'enkephalinase' identical with 'angiotensin-converting enzymes'?" page 204, column 2, abstract no. 163896u**

(73) Proprietor: **THE WELLCOME FOUNDATION LIMITED**
**183-193 Euston Road**
**London NW1 2BP (GB)**

(72) Inventor: **Wilkinson, Samuel**
**12 Bevington Road**
**Beckenham Kent (GB)**

(74) Representative: **Berg, Wilhelm, Dr. et al,**
**Dr. Berg, Dipl.-Ing. Stapf, Dipl.-Ing. Schwabe, Dr. Dr. Sandmair Mauerkircherstrasse 45**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Pharmaceutical amides, and preparation, formulations and use thereof

This invention relates to amides and their preparation, to pharmaceutical formulations containing such compounds and the preparation of such formulations, to the use of such compounds in human and verterinary medicine, and to intermediates of value in the preparation of the amides and the preparation of such intermediates.

In 1975, Hughes *et al.* (*Nature* Vol. 258, December 18, 1975 pages 577 to 579) identified two related pentapeptides from the mammalian brain with potent opiate agonist activity, the enkephalins:

H.Tyr.Gly.Gly.Phe.Met.OH (Met$^5$-enkephalin)

H.Tyr.Gly.Gly.Phe.Leu.OH (Leu$^5$-enkephalin)

(The abbreviations used herein for amino acids and their radicals are those conventional in the art and may be found in, for example, *Biochemical Journal* (1972) 126, pages 773 to 780. In the above and throughout the following all references are to the *L*-configuration of chiral amino acids and their radicals unless otherwise stated).

Since this discovery the enkephalins have been studied by a number of workers and from a variety of approaches. One such approach concerns investigation of their inactivation and recent reports (for example Malfroy *et al., Nature* Vol. 276, 30 November 1978 pages 523 to 526 and Gorenstein *et al., Life Sciences* Vol. 25 (1979) pages 2065 to 2070) have indicated that there exists in mammalian brain a dipeptidylcarboxypeptidase ("enkephalinase") capable of hydrolysing the Gly$^3$-Phe$^4$ bond

↓

H.Tyr.Gly.Gly.Phe$^{Met}_{Leu}$.OH

and thus generating the biologically inactive N-terminal tripeptide fragment

H.Tyr.Gly.Gly.OH

Enkephalinase thus has a role in some ways comparable with that of the mammalian angiotensin converting enzyme (ACE, EC 3.4.15.1) which acts upon the relatively inactive decapeptide angiotensin I

H.Asp.Arg.Val.Tyr.$^{Val}_{Ile}$.His.Pro.Phe.His.Leu.OH.

at the Phe$^8$-His$^9$ bond to release the potent pressor octapeptide angiotensin II

H.Asp.Arg.Val.Tyr.$^{Val}_{Ile}$.His.Pro.Phe.OH

although it has been demonstrated (Swerts *et al., European Journal of Pharmacology* Vol. 57 (1979) pages 279 to 281) that the two enzymes are distinct species.

Controlling the liberation of angiotensin II from angiotensin I, by selectively inhibiting ACE, has for some time been regarded as a possible method for the therapy of hypertension and a number of agents, originating from such an approach and exhibiting the desired properties, have been described. One especially potent compound is 1 - (*D* - 3 - mercapto - 2 - methylpropanoyl) - *L* - proline (R, S), otherwise known as captopril or SQ 14 225 and having the structure

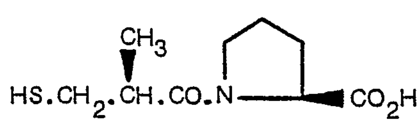

This has been reported as capable of inhibiting both enkephalinase and ACE (Swerts *et al., loc. cit.)* but as having a far greater specifity for the latter enzyme than for the former, the concentration of compound required to inhibit ACE by 50% being approximately 1000-fold lower than that required to effect the same degree of inhibition of enkephalinase.

The present invention relates to a class of compounds which have not only an advantageous

enkephalinase inhibitory activity but also, in distinction to SQ 14 225, a greater specificity for enkephalinase than for ACE.

The present invention thus provides the amides of formula (I)

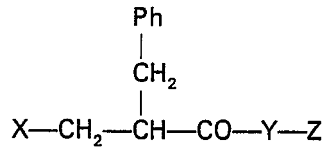

(I)

together with basic salts thereof (i.e. salts formed by reaction of a compound of formula (I) with a base), wherein

X is a group capable of functioning as a ligand for a zinc ion;

Ph is a phenyl group which is optionally substituted by one or more substituents selected from halo (i.e. fluoro, chloro, bromo, or iodo) and nitro;

Y is a group of formula:—

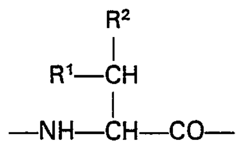

where

$R^1$ is hydrogen or methyl;

$R^2$ is alkyl of 1 to 3 carbon atoms or is methyl thiomethyl; and

Z is $—OR^3$ or $—NR^4R^5$ where $R^3$, $R^4$ and $R^5$ are each hydrogen or alkyl of 1 to 4 carbon atoms and $R^3$ can further be phenylalkyl having 1 to 3 carbon atoms in the alkylene moiety thereof or phenyl.

Formula (I) as above defined includes a plurality of asymmetric centres and should be understood to include all optical isomers embraced thereby and mixtures thereof.

Suitable identities for the group X include alkanoylthio having 2 to 5 carbon atoms, benzoylthio, phenylalkanoylthio having 1 to 3 carbon atoms in the alkylene moiety thereof, carboxyl, formyl, hydroxyamino, mercapto, phosphono and (SH)monothiophosphono, i.e. OH(SH).O.P—.

In the salts of the amides of formula (I) the pharmacological activity resides in the amide (acid) anion and the identity of the cation is of less importance although for therapeutic purposes it is preferably pharmacologically and pharmaceutically acceptable to the recipient. Acceptable salts include ammonium salts, alkali metal salts such as sodium and potassium salts, alkaline earth metal salts such as magnesium and calcium salts, and salts of organic bases, for example amine salts derived from mono-, di- or trilower alkylamines or cycloalkylamines such as dicyclohexylamine or alkanolamines such triethanolamine and diethylaminoethylamine and salts with heterocyclic amines such as piperidine, pyridine, piperazine and morpholine.

As subclasses of the amides of formula (I) may be mentioned those compounds wherein

(i) X is carboxy, alkanoylthio (for example acetylthio) or mercapto;

(ii) Ph is unsubstituted phenyl;

(iii) Y is a group (in either the *D*- or *L*-configuration) of formula:—

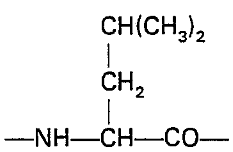 or 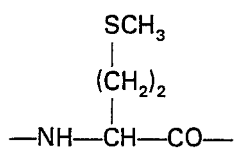

(iv) Z is —OH or $—NH_2$.

The amides of formula (I) and their basic salts may be prepared by any of the methods known in the art for the preparation of compounds of analogous structure. Thus they may be prepared by reacting a reagent (II) with a reagent (III)

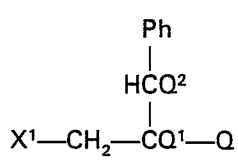

(II)

$$H—Y—Z^1 \qquad (III)$$

wherein

Ph and Y are as defined in formula (I);

$X^1$ is a group X as defined in formula (I) or a functionally protected derivative thereof;

$Z^1$ is a group Z as defined in formula (I) or a functionally protected derivative thereof;

Q is carboxyl or a functionally activated derivative thereof; and

$Q^1$ and $Q^2$ are both hydrogen or together form a bond;

followed (when $Q^1$ and $Q^2$ together form a bond) by selective reduction of the said bond and, as appropriate, by deprotection of the product and conversion of the product into the amide or a basic salt thereof.

The reaction of (II) with (III) may be effected using techniques standard in peptide chemistry and using either classical methods of peptide synthesis or solid phase procedures. Details of suitable activating and protecting groups and of suitable reaction conditions (both for the reaction of (II) with (III) and for the removal of protecting groups) may be found in the following literature which is given purely by way of exemplification and which is intended to be neither exhaustive nor limiting:

a) Schröder and Lüebke, *"The Peptides"* (Academic Press) (1965).

b) Bellean and Malek, *J. Am. Chem. Soc., 90,* 165 (1968).

c) Tilak, *Tetrahedron Letters*, 849 (1970).

d) Beyerman, *Helv. Chim. Acta., 56,* 1729 (1973).

e) Stewart and Young, *"Solid Phase Peptide Synthesis"* (W. H. Freeman and Co.) (1969).

Certain of the amides of formula (I) may also be prepared from precursors which are themselves within formula (I).

Thus,

(i) compounds wherein Z is —$OR^3$ where $R^3$ is hydrogen may be prepared by hydrolysis of corresponding compounds where $R^3$ is alkyl, phenylalkyl or phenyl;

(ii) compounds wherein Z is —$OR^3$ where $R^3$ is alkyl, phenylalkyl or phenyl may be prepared by esterification of the corresponding compound where $R^3$ is hydrogen;

(iii) compounds wherein Z is —$NR^4R^5$ may be prepared by reaction of a corresponding compound wherein Z is —$OR^3$ where $R^3$ is alkyl, phenylalkyl or phenyl with as appropriate ammonia or a mono- or dialkylamine;

(iv) compounds wherein X is mercapto may be prepared from corresponding compounds wherein X is alkanoylthio, benzoylthio or phenylalkanoylthio by treatment with an agent such as methanolic ammonia solution.

The amides of formula (I) may be converted into basic salts thereof, and the converse, by well established techniques.

In the preparation of certain of the amides of formula (I) and basic salts thereof wherein X is carboxyl a convenient reagent (II) is a *D* - (+) - 2 - benzyl - 3 - carbethoxypropionic acid ((IV), Ph is as defined in formula (I)). This may be obtained by, for example, resolving the corresponding *DL* - diethyl - 2 - benzylsuccinate (V) with chymotrypsin according to the procedure of Cohen *et al., Journal of the American Chemical Society* (1968) *90*, 3495, the enzyme selectively revealing the required carboxyl group.

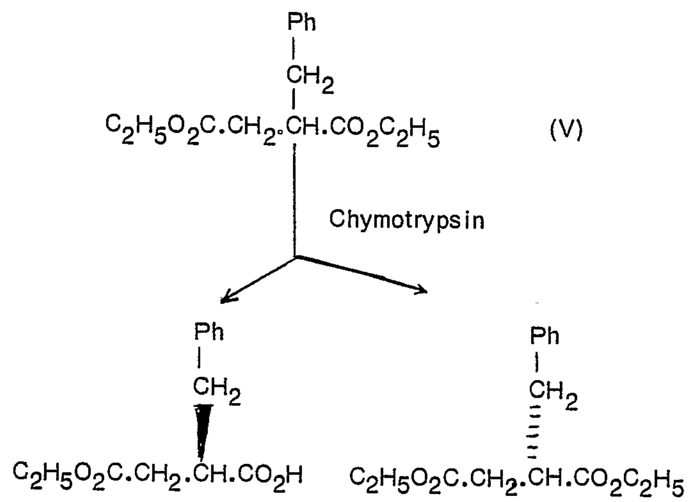


(V)

(IV)

In an alternative procedure the reagent (II) is a 3 - benzyloxycarbonyl - 2 - benzylidene-propionic acid ((VI), Ph is as defined in formula (I) and Bzl is benzyl) which may be prepared by

preferentially opening the corresponding benzylidene succinic anhydride (VII) with benzyl alcohol according to the procedure of Cohen *et al., ibid.* to again provide the required carboxyl group.

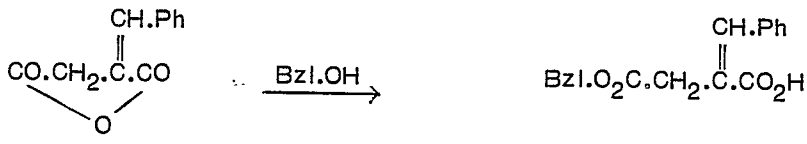


(VII) (VI)

The acid (VI) is reacted with a reagent (III) as hereinabove defined to provide (VIII) and the double bond in the latter reduced by, for example, hydrogenation in the presence of palladium charcoal. The hydrogenation will also effect conversion of the benzyloxycarbonyl group to the free (deprotected) carboxyl and it is convenient (where the desired amide of formula (I) has Z as hydroxyl) for $Z^1$ in (III) to be benzyloxy as the latter group will be similarly deprotected.

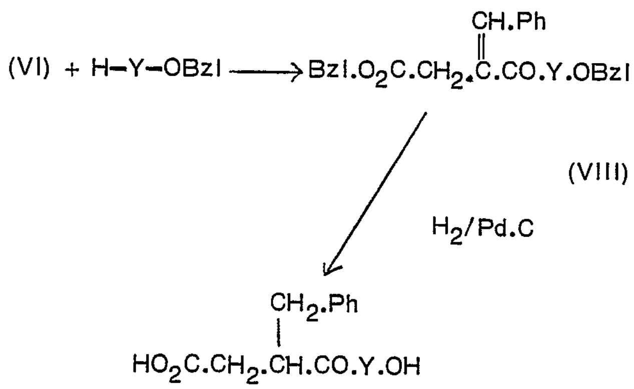


(VIII)

When the preparative procedures hereinabove described provide a mixture of optical isomers of the amide of formula (I) or of an intermediate thereto, for example a mixture of diastereoisomers, the individual isomers may be separated by appropriate conventional physical techniques such as high performance liquid chromatography, preparative thin layer chromatography and the like.

Because of their selective enkephalinase-inhibiting activity the amides of formula (I) and the basic salts thereof are of value in the *in vitro* and *in vivo* investigation of the mode of action and role of the enzyme and in its localization, isolation and purification.

For example, the present invention provides a method which comprises contacting an amide of formula (I) or a basic salt thereof with enkephalinase and determining the inhibitory effect of the amide or salt on the enzyme activity of the enkephalinase. This method can be used to compare the enkephalinase inhibitory effect of the above compounds with other compounds having a similar effect. The compounds according to the invention can be radiolabelled, if desired, to facilitate the determination of their inhibitory effect.

Their selective enkephalinase-inhibiting activity also confers on the amides of formula (I) and the pharmacologically and pharmaceutically acceptable basic salts thereof utility in the prolongation and/or potentiation in a mammal of the effects of enkephalins of either endogenous or exogenous origin including in the later case synthetic enkephalin analogues. The said amides and salts thus have the same activities and utilities as have been indicated for the endogenous compounds.

In particular, the amides of formula (I) and the pharmacologically and pharmaceutically acceptable basic salts thereof have morphinomimetic (morphine agonist) activity and thus may be used in the treatment of mammals in the fields of both human and veterinary medicine in any condition where an agent with a morphine-like effect is indicated.

The pharmacological properties and therapeutic uses of morphine are well documented in the literature (see for example *'The Pharmacological Basis of Therapeutics'*, Goodman, L. S. and Gilman, A eds., published by Macmillan Publishing Co., Inc., New York, fifth edition (1975), ISBN 0—02—344781—8, especially at Chapter 15 pages 245 to 283, and *'Martindale: The Extra Pharmacopoeia'*, Wade, A ed., published by The Pharmaceutical Press, London, twenty-seventh edition (1977), ISBN 0—85369—114—2, especially at pages 970 to 974) and specific utilities for the said amides and salts include, by way of example, the following.

(1) The relief of pain (analgesia), for example pain arising from spasm of smooth muscle as in

renal or biliary colic, pain in terminal illness such as terminal cancer, pain in the postoperative period, and obstetrical pain.

(2) The induction of constipation, for example after ileostomy or colostomy.

(3) The treatment of diarrhoea or dysentery.

(4) The suppression of cough.

(5) The induction of sleep, especially where sleeplessness is due to pain or cough.

(6) Sedation, for example in pre-anaesthetic medication to reduce preoperative apprehension.

(7) Tranquillization, for example when applied to the relief of pain in terminal illness such as terminal cancer, and the relief of anxiety in general.

(8) The induction of euphoria and the treatment of depression, for example when applied to the relief of pain in terminal illness such as terminal cancer.

(9) The relief of dyspnoea, for example that of acute left ventricular failure or pulmonary oedema.

The amides of formula (I) and the pharmacologically and pharmaceutically acceptable basic salts thereof (hereafter collectively referred to as the active ingredients) may be administered to the human or non-human recipient by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general however, for each of these utilities and indications, a suitable, effective dose will be in the range 0.075 $\mu$g to 12 mg per kilogram bodyweight of recipient (human or non-human) per day, preferably in the range 0.75 $\mu$g to 1.2 mg per kilogram bodyweight per day and most preferably in the range 7.5 to 120 $\mu$g per kilogram bodyweight per day; an optimum dose is 30 $\mu$g per kilogram bodyweight per day. (Unless otherwise indicated all weights of active ingredient are calculated as the amide of formula (I): for salts thereof the figures would be increased proportionately.) The desired dose is preferably presented as between two and four sub-doses administered at appropriate intervals throughout the day. Thus where three sub-doses are employed each will generally lie in the range 0.025 $\mu$g to 4 mg, preferably 0.25 $\mu$g to 0.4 mg and most preferably 2.5 to 40 $\mu$g per kilogram bodyweight with an optimum of 10 $\mu$g per kilogram bodyweight. A daily dose for a human weighing of the order of 50 kg will thus generally lie in the range 3.75 $\mu$g to 600 mg, preferably in the range 37.5 $\mu$g to 60 mg and most preferably in the range 0.375 to 6.0 mg and may conveniently be presented as three equal unit sub-doses of 1.25 $\mu$g to 200 mg, preferably 12.5 $\mu$g to 20 mg and most preferably 0.125 to 2.0 mg. Optimally a human daily dose, for an individual weighing of the order of 50 kg, is 1.5 mg conveniently presented as three unit sub-doses each of 0.5 mg.

While it is possible for the active ingredients to be administered as the raw chemical it is preferable to present them as a pharmaceutical formulation preparation. The formulations, both veterinary and for human use, of the present invention comprise an active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous), intramuscular, intravenous, intradermal, intrathecal and epidural) administration. The formulations may conveniently be presented in unit dosage form and may be prepared by any of the methods well known in the art of pharmacy. All methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for rectal administration may be presented as a suppository with the usual carriers such as cocoa butter.

Formulations suitable for nasal administration wherein the carrier is a solid include a coarse

powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for topical administration in the mouth include lozenges comprising the active ingredient in a flavoured basis, usually sucrose and acacia or tragacanth; and pastilles comprising the active ingredient in an inert basis such as gelatin and glycerin, or sucrose and acacia.

Formulations suitable for vaginal administration may be presented as pessaries, creams, pastes or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as hereinabove recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

All references identified hereinabove or in the following are hereby incorporated herein by reference thereto.

Those basic salts which are not pharmacologically and pharmaceutically acceptable may be converted to the amides themselves and to salts thereof which are acceptable by standard procedures.

It will be understood from the foregoing description that this invention may comprise any novel feature described herein, principally but not exclusively for example:

(a) Amides of formula (I) as hereinbefore defined and the basic salts thereof.

(b) Methods as hereinbefore described for the preparation of compounds according to (a) *supra*, together with the compounds when so prepared.

(c) A pharmaceutical formulation comprising a therapeutically effective amount of an amide of formula (I) as hereinbefore defined or a pharmacologically and pharmaceutically acceptable basic salt thereof together with an acceptable carrier therefor.

(d) A method for the preparation of a formulation according to (c) *supra* comprising admixture of the active ingredient, as defined, with the carrier therefor.

(e) Amides of formula (I) as hereinbefore defined and pharmacologically and pharmaceutically acceptable basic salts thereof, for use in the therapeutic treatment of a mammal.

(f) Amides of formula (I) as hereinbefore defined and pharmacologically and pharmaceutically acceptable basic salts thereof, for use in the therapeutic treatment of a human.

(g) Amides of formula (I) as hereinbefore defined and pharmacologically and pharmaceutically acceptable basic salts thereof, for use in prolongation and/or potentiation in a mammal of the effects of endogenous or exogenous enkephalins.

(h) Amides of formula (I) as hereinbefore defined and pharmacologically and pharmaceutically acceptable basic salts thereof, for use in the treatment of a mammal for a condition where an agent with a morphine-like effect is indicated.

(i) Amides of formula (I) as hereinbefore defined and pharmacologically and pharmaceutically acceptable basic salts thereof, for use in the treatment of a mammal for a condition selected from those specifically identified hereinabove under (1), (2), (3), (4), (5), (6), (7), (8) or (9).

(j) A method for the prolongation and/or potentiation in a mammal of the effects of endogenous or exogenous enkephalins comprising administration to the mammal of a non-toxic therapeutically effective amount of an amide of formula (I) as hereinbefore defined or a pharmacologically and pharmaceutically acceptable basic salt thereof.

(k) A method for the treatment of a mammal for a condition where an agent with a morphine-like effect is indicated comprising administration to the mammal of a non-toxic, therapeutically effective amount of an amide of formula (I) as hereinbefore defined or a pharmacologically and pharmaceutically acceptable basic salt thereof.

(l) A method for the treatment of a mammal for a condition selected from those specifically identified hereinabove under (1), (2), (3), (4), (5), (6), (7), (8) or (9) comprising administration to the mammal of a non-toxic, therapeutically effective amount of an amide of formula (I) as hereinbefore defined or a pharmacologically and pharmaceutically acceptable basic salt thereof.

(m) A method according to (j), (k) or (l) *supra* wherein the mammal is man.

(n) Novel compounds of formulae (II), (III), (IV), (V), (VI), (VII) and (VIII) as hereinbefore defined.

The following Examples are provided in illustration of the present invention and should not be construed as in any way constituting a limitation thereof. All temperatures are in degrees Celsius.

Example 1

(2*S*)-2-[(2*RS*)-2-Benzyl-3-carboxypropanamido]-4-methylpentanoic acid (compound 1)

Step (a): N-[DL-2-benzyl-3-carbethoxypropanoyl]-L-leucine methyl ester

The above ester was prepared by condensing (+)-2-benzyl-3-carbethoxypropionic acid (5.07 g) with L-leucine methyl ester hydrochloride (3.90 g) in dimethylformamide (50 ml) in the presence of 1-hydroxybenzotriazole (5.80 g), dicyclohexylcarbodiimide (4.43 g) and triethylamine (2.97 ml) according to customary procedures. The product was an oil (6.85 g).

Step (b): (2*S*)-2-[(2*RS*)-2-Benzyl-3-carboxypropanamido]-4-methylpentanoic acid

The oil from step (a) (6.85 g) was dissolved in methanol (75 ml) and water (8 ml) and saponified (pH stat) at pH 12.0 with N sodium hydroxide. After the addition of 41.5 ml of alkali, the mixture was concentrated *in vacuo* to remove the methanol, diluted with 25 ml water and filtered. The filtrate was cooled to 0°C and acidified by the addition of 2N hydrochloric acid (20.8 ml) and extracted twice with ethyl acetate (150 ml). The combined extracts were washed with 50% saturated sodium chloride solution (25 ml), dried and evaporated. The residue was triturated with ether, the solid filtered and crystallised from aqueous ethanol as colourless prisms, m.pt. 165°—167°C; $[\alpha]_D^{23} = -22.7°$, c = 1.0 in ethanol; Rf 0.35[1] and 0.66[1], 0.62[2].

Analysis $C_{17}H_{23}NO_5$ requires C, 63.55; H, 7.17; N, 4.36%
found C, 63.63; H, 7.15; N, 4.43%

Example 2

(2*S*)-2-[(2*RS*)-2-Benzyl-3-carboxypropanamido]-4-methyl-pentanoic acid (compound 1)

Step (a): N-[3-Benzoyloxycarbonyl-2-benzylidenepropanoyl]-L-leucine benzyl ester

To a solution of 3-benzyloxycarbonyl-2-benzylidene-propionic acid (8.88 g) in dimethylformamide (100 ml) cooled to −10°C was added 1-hydroxybenzotriazole (8.10 g) and dicyclohexylcarbodiimide (6.18 g). After stirring for 15 minutes L-leucine benzyl ester hydrochloride (6.73 g) and triethylamine (4.15 g) were added and stirring continued at +4°C for 24 hours. The dicyclohexylurea was removed by filtration and the filtrate evaporated *in vacuo*. The residue was dissolved in ethyl acetate and acid/base washed in the usual manner, dried and concentrated *in vacuo*, to give an oil (14 g).

Step (b): (2*S*)-2-[(2*RS*)-2-Benzyl-3-carboxypropanamido]-4-methylpentanoic acid

The oil from step (a) (14 g) was hydrogenated in methanol (250 ml) in the presence of 10% (w/w) palladium on charcoal catalyst (2 g). When the uptake of hydrogen ceased, the catalyst was filtered and the filtrate concentrated *in vacuo* to give a residue which solidified on trituration with ether and was found to be identical with the product of Example 1. $[\alpha]_D^{18} = -24.6°$, c = 1.0 in methanol; Rf 0.35[1] and 0.66[1], the two spots of equal intensity when located with iodine/starch reagent.

Example 3

The product of Example 2 was fractionated on a Waters Associates Preparative LC (System 500) using silica gel cartridges (Preppak 500) and the solvent system methylene dichloride containing methanol (4%) and glacial acetic acid (1%). The components were identified by acid hydrolysis to their constituents as:

(2*S*)-2-[(2*R*)-2-benzyl-3-carboxypropanamido]-4-methylpentanoic acid (compound 2); $[\alpha]_D^{18} = +24.8°$, c = 1.0 in methanol; Rf 0.66[1]; m.pt. 139—140°C, yielding L-leucine and (+)-benzylsuccinic acid; and

(2*S*)-2-[(2*S*)-2-benzyl-3-carboxypropanamido]-4-methylpentanoic acid (compound 3); $[\alpha]_D^{18} = -74.5°$, c = 1.0 in methanol; Rf 0.36[1]; m.pt. 199—201°C, yielding L-leucine and (−)-benzylsuccinic acid.

Example 4

(2*S*)-2-[(2*R*)-2-Benzyl-3-carboxypropanamido]-4-methyl-pentanamide (compound 4)

Step (a): N-[D-2-benzyl-3-carbethoxypropanoyl]-L-leucinamide

The above amide was prepared by standard procedures from (+)-2-benzyl-3-carbethoxypropionic acid (4.62 g), L-leucinamide hydrochloride (3.26 g), 1-hydroxybenzotriazole (5.29 g), dicyclohexylcarbodiimide (4.03 g) and triethylamine (2.71 ml) in dimethylformamide (50 ml). The product was isolated as an amorphous solid (3.71 g) by precipitation from isopropanol with diisopropyl ether. Rf 0.44[4], 0.55 (methylethylketone).

Analysis $C_{19}H_{28}N_2O_4$ requires C, 65.51; H, 8.05; N, 8.05%
found C, 65.14; H, 8.41; N, 8.16%

Step (b): (2S)-2-[(2R)-2-Benzyl-3-carboxypropanamido]-4-methylpentanamide

The product of step (a) (1.74 g) in methanol (45 ml) and water (5 ml) was saponified using N sodium hydroxide (pH stat 12.0). The product obtained after customary procedures crystallised from aqueous methanol in colourless prisms, m.pt. 214—215°C; $[\alpha]_D^{23} = -86.9°$, c = 0.5 in methanol; Rf 0.58[1].

Analysis $C_{17}H_{24}N_2O_4$ requires C, 63.75; H, 7.50; N, 8.75%
found C, 63.89; H, 7.68; N, 8.80%

Example 5

t-butyl-(2*S*)-2-[(2*RS*)-3-Acetylthio-2-benzylpropanamido]-4-methylpentanoate (compound 5)

Step (a): DL-3-Acetylthio-2-benzylpropionic acid

2-Benzylacrylic acid (16.2 g) (prepared according to the procedure of C. Mannich and K. Risert, *Ber.* (1924) *57*, 1116, from diethyl benzylmalonate) was stirred at ambient temperature for 1 hour with thiolacetic acid (10 ml). The mixture was then heated on a steam bath for 1 hour, concentrated under reduced pressure and then re-evaporated three times with benzene (50 ml).

The residue was dissolved in a mixture of dry ether (20 ml) and hexane (50 ml) and redistilled dicyclohexylamine (19.5 ml) in hexane (50 ml) added. The crystalline dicyclohexylamine salt (33.5 g) was filtered and washed with hexane, m.pt. 102—103°C.

Analysis $C_{24}H_{37}NO_3S$ requires C, 68.74; H, 8.83; N, 3.34%
found C, 68.85; H, 8.99; N, 3.13%

The salt was suspended in ethyl acetate (200 ml) and stirred vigorously with potassium bisulphate (16 g) in water (50 ml). The ethyl acetate phase was separated, washed with water (50 ml), dried (anhydrous sodium sulphate) and concentrated *in vacuo* to give the product acid as an oil (19.8 g).

Step (b): t-butyl(2*S*)-2-[(2*RS*)-3-Acetylthio-2-benzylpropanamido]-4-methylpentanoate

DL-3-Acetylthio-2-benzylpropionic acid (5.39 g) was dissolved in methylene dichloride (75 ml) and cooled to −10°C. 1-Hydroxybenzotriazole (6.11 g) and dicyclohexylcarbodiimide (4.66 g) were added. After stirring for 20 minutes, L-leucine tert.butyl ester (4.24 g) were added and stirring continued for 24 hours at +4°C. The dicyclohexylurea was filtered and the filtrate concentrated *in vacuo*. The residue was dissolved in ethyl acetate (50 ml) and after refrigeration additional urea removed by filtration. The filtrate was diluted to 350 ml with ethyl acetate and washed successively with 50 ml portions of 5% citric acid, 50%-saturated sodium chloride, saturated sodium bicarbonate and 50%-saturated sodium chloride solutions. The solution was dried (anhydrous sodium sulphate) and concentrated *in vacuo* to give a crystalline solid which was recrystallised from light petroleum, m.pt. 80—81°C.

Example 6

(2*S*)-2-[(2*RS*)-3-Acetylthio-2-benzylpropanamido]-4-methylpentanoic acid, dicyclohexylamine salt (compound 6)

The t-butyl ester product of Example 5 (9 g) was stirred at ambient temperature for one hour with redistilled trifluoroacetic acid (100 ml) and anisole (50 ml). The mixture was concentrated *in vacuo* and the residue partitioned between saturated sodium bicarbonate solution (100 ml) and ethyl acetate (250 ml). The aqueous phase was acidified and extracted twice with ethyl acetate (150 ml). The combined extracts were washed with 50%-saturated sodium chloride solution, dried (anhydrous sodium sulphate) and concentrated *in vacuo*. The residual gum (6.5 g) was dissolved in ethyl acetate (30 ml) and redistilled dicyclohexylamine (4.4 ml) added. The crystalline salt was filtered, washed with cold ethyl acetate and recrystallised from acetonitrile as colourless prisms, m.pt. 144—146°C; $[\alpha]_D^{19} = -16.58°$, c = 1.0 in methanol; Rf 0.68[1].

Analysis $C_{30}H_{48}N_2O_4S$, $1.5H_2O$ requires C, 64.40; H, 9.12; N, 5.00%
found C, 64.74; H, 9.00; N, 4.88%

Example 7

(2*S*)-2-[(2*RS*)-2-Benzyl-3-mercaptopropanamido]-4-methylpentanoic acid, dicyclohexylamine salt (compound 7).

The dicyclohexylamine salt product of Example 6 (6.2 g) was suspended in ethyl acetate (100 ml) and stirred vigorously with potassium bisulphate (2.1 g) in water (20 ml). The ethyl acetate phase was washed with water (25 ml), dried (anhydrous sodium sulphate) and concentrated *in vacuo*.

The residue (4.1 g) was stirred under argon for 2 hours with 5.5N ammonia in methanol (50 ml). The solution was concentrated *in vacuo*. The residue was dissolved in ethyl acetate (100 ml) and shaken successively with 15 ml portions of 5% aqueous citric acid and 50%-saturated sodium chloride solutions. The solution after drying was concentrated *in vacuo*. The residue was dissolved in ether (25 ml) under argon and redistilled dicyclohexylamine (1.3 ml) added. The crystalline dicyclohexylamine salt was filtered as colourless prisms and washed with ether and light petroleum, m.pt. 154—155°C.; $[\alpha]_D^{19} = -34.2°$, c = 0.7 in methanol; Rf 0.68[1].

Analysis $C_{28}H_{46}N_2O_3S$ requires C, 68.57; H, 9.39; N, 5.71%
found C, 68.19; H, 9.17; N, 5.61%

The product gave positive reactions for —SH with alkaline sodium nitroprusside and with sodium azide/iodine solutions.

Example 8

(2*S*)-2-[(2*RS*)-3-Acetylthio-2-benzylpropanamido]-4-methylpentanamide (compound 8)

A mixture of DL-3-acetylthio-2-benzylpropionic acid (Example 5, step (a)) (4.82 g), 1-hydroxybenzotriazole (5.47 g) and dicyclohexylcarbodiimide (4.17 g) were stirred in dimethylformamide (60 ml) for 30 mins. at −10°C. L-Leucinamide hydrochloride (3.37 g) and triethylamine (2.80 ml) were added and stirring continued at +4°C for 24 hours. The dicyclohexylurea was filtered and the filtrate concentrated *in vacuo*. The residue was dissolved in ethyl acetate and acid/base washed in the usual manner. Evaporation of the solvent and recrystallisation of the residue from isopropanol/diisopropyl ether gave colourless prisms, m.pt. 128—130°C.

Analysis $C_{30}H_{49}N_3O_3S$ requires C, 61.71; H, 7.43; N, 8.00%
found C, 61.85; H, 7.27; N, 8.35%

Example 9

(2*S*)-2-[(2*RS*)-2-Benzyl-3-mercaptopropanamido]-4-methyl -pentanamide (compound 9)

The product of Example 8 (3.65 g) was stirred for 2 hours under argon with 5.5N ammonia in methanol (50 ml). The mixture was concentrated *in vacuo*, the residue triturated with water and the solid filtered and recrystallised from aqueous isopropanol as colourless needles, m.pt. 145°C; $[\alpha_D^{20} = -45.1°$, c = 1.0 in methanol; Rf 0.30[4].

Analysis $C_{16}H_{24}N_2O_2S,0.5H_2O$
requires C, 60.57; H, 7.89; N, 8.83%
found C, 60.44; H, 7.45; N, 8.38%

Example 10

(2*R*)-2-[(2*R*)-2-Benzyl-3-carboxypropanamido]-4-methylpentanamide (compound 10)

This was prepared in an analogous manner to the corresponding (2*S*)-2-(2*R*) compound (4, Example 4), using instead D-leucinamide hydrochloride in the first step.

The product was obtained as colourless prisms, m.pt. 212—214°C; $[\alpha]_D^{24} = +94.3°$, C = 0.5 in methanol; Rf 0.58[1], 0.48[2].

Analysis $C_{17}H_{24}N_2O_4$ requires C, 63.75; H, 7.50; N, 8.75%
found C, 63.58; H, 7.20; N, 8.60%

Example 11

By analogous procedures to those set out in Example 1, substituting for the L-leucine methyl ester hydrochloride in step (a) therein respectively D-leucine methyl ester hydrochloride, D-methionine methyl ester hydrochloride and L-methionine methyl ester hydrochloride, there were prepared the following:

(2*R*)-2-[(2*RS*)-2-Benzyl-3-carboxypropanamido]-4-methylpentanoic acid (compound 11); colourless prisms, m.pt. 164—166°C; $[\alpha]_D^{22} = +25.7°$, C = 0.5 in methanol; Rf 0.35[1] and 0.66[1], 0.21[2].

Analysis $C_{17}H_{23}NO_5$ requires C, 63.55; H, 7.17; N, 4.36%
found C, 63.42; H, 7.12; N, 4.43%

(2*R*)-2-[(2*RS*)-2-Benzyl-3-carboxypropanamido]-4-(methylthio)butanoic acid (compound 12); colourless prisms, m.pt. 131—134°C (decomp.); $[\alpha]_D^{21} = +14.9°$, c = 1.0 in methanol; Rf 0.35[1] and 0.43[1], 0.51[2], 0.63[3].

Analysis $C_{16}H_{21}NO_5S$ requires C, 56.64; H, 6.19; N, 4.13%
found C, 56.61; H, 6.24; N, 4.13%

(2*S*)-2-[(2*RS*)-2-Benzyl-3-carboxypropanamido]-4-(methylthio)butanoic acid (compound 13); colourless prisms, m.pt. 137—139°C; $[\alpha]_D^{21} = -7.48°$ c = 0.5 in methanol; Rf 0.35[1] and 0.43[1]; 0.51[2]; 0.69[3].

Analysis $C_{16}H_{21}NO_5S$ requires C, 56.64; H, 6.19; N, 4.13%
found C, 56.61; H, 6.26; N, 4.15%

## Example 12

(2*S*)-2-[(2*RS*)-3-Carboxy-2-(4-nitrobenzyl)propanamido]-4-methylpentanoic acid (compound 14)

Diethyl (*p*-nitrobenzyl)malonate, prepared by treating diethyl benzylmalonate with fuming nitric acid, was converted to *p*-nitrobenzylsuccinic acid and thence to diethyl (*p*-nitrobenzyl)succinate by conventional procedures and the latter diester resolved with chymotrypsin according to the method of Cohen *et al., J.A.C.S.* (1968) *90,* 3495. The (+) - 3 - carbethoxy - 2 - (4 - nitrobenzyl)propionic acid thereby obtained was reacted with L-leucine methyl ester hydrochloride in the usual manner in the presence of 1-hydroxybenzotriazole and dicyclohexylcarbodiimide and the resulting protected product saponified with N sodium hydroxide to yield the title compound.

Colourless prisms, m.pt. 143—145°C; $[\alpha]_D^{20} = -23.6°$, c = 0.5 in methanol; Rf 0.30[1] and 0.67[1], 0.46[2]

Analysis $C_{17}H_{22}N_2O_7$ requires C, 55.74; H, 6.01; N, 7.65%
found C, 55.97; H, 6.28; N, 7.99%

## Example 13

(2*R*)-2-[2*RS*-2-Benzyl-3-mercaptopropanamido]-4-(methylthio)butanoic acid (compound 15)

Step (a) t-Butyl(2R)-2-[2*RS*-3-acetylthio-2-benzylpropanamido]-4-(methylthio)butanoate

3-Acetylthio-2-benzylpropionic acid (4.75 g) was dissolved in methylene dichloride (75 ml) cooled to −10°C and 1-hydroxybenzotriazole (5.4 g) and dicyclohexylcarbodiimide (4.11 g) added. After 1 hour, D-methionine *tert*-butyl ester (4.09 g) was added and stirring continued at +4°C overnight. The mixture was filtered and the filtrate concentrated *in vacuo.* The residue was dissolved in ethyl acetate (350 ml) and washed successively with 50 ml portions of 50% saturated sodium chloride solution; 5% citric acid solution; saturated sodium bicarbonate solution; 50% saturated sodium chloride solution. The solution was dried ($MgSO_4$) and concentrated to an oil (8.0 g) *in vacuo.* Rf 0.82[5]; 0.79[4]; 0.88[6].

Step (b) (2R)-2-[2*RS*-2-Acetylthio-2-benzylpropanamido]-4-(methylthio)butanoic acid

The oil obtained in step (a) was dissolved in anisole (50 ml), trifluoroacetic acid (100 ml) added and the solution stirred at ambient temperature for 2 hours. The mixture was concentrated *in vacuo* to give an oily residue (6.97 g). Rf 0.76[6].

Step (c) (2R)-2-[2RS-2-Benzyl-3-mercaptopropanamido]-4-(methylthio)butanoic acid

The oil residue obtained in step (b) was stirred at ambient temperature under argon for 2 hours with 5.08N ammonia in methanol (75 ml). The solution was evaporated *in vacuo* and the residue dissolved in ethyl acetate (125 ml), washed with 15 ml portions of 5% citric acid solution and 50% saturated sodium chloride solution, dried ($MgSO_4$) and concentrated *in vacuo* to an oil.

The oil (5.4 g) was dissolved in dry ether (50 ml) and dicyclohexylamine (3.2 ml) added. The precipitate was filtered and dried, m.pt. 155°C after sintering at 145°C; $[\alpha]_D^{21} = +7.9°$ (c = 0.5 in methanol); Rf 0.70[6].

Analysis $C_{27}H_{44}N_2O_3S_2$ requires C, 63.78; H, 8.66; N, 5.51%
found C, 63.86; H, 8.,94; N, 5.50%

In the foregoing Examples, the Rf figures refer to thin layer chromatography using Merck silica gel plates and the following solvent systems:

1) chloroform: methanol: 5% (v/v) acetic acid (120:90:5)
2) chloroform: methanol: 5% ammonia (120:90:5)
3) *n*-butanol: acetic acid: water (3:1:1)
4) chloroform: methanol (8:1)
5) methyl ethyl ketone
6) chloroform: methanol: 32% acetic acid (120:90:5)

Activity in Vitro

The compounds were investigated for enkephalinase-inhibiting activity using the following methods.

A) Purified enkephalinase A1 was obtained according to the following procedure (modification of the method of Gorenstein and Snyder, Life Sciences, Vol. 25, pages 2065—2070, 1979).

Rats were killed by decapitation and the striata dissected out on ice. The pooled tissues were homogenised in ice-cold Tris/hydrochloric acid buffer (50 mM, pH 7.70, 30 ml per gram of tissue) and centrifuged (50,000 g, 15 mins). The resulting supernatant was discarded and the remaining pellet washed three times. The washed pellet was solubilised by resuspension in half the volume of buffer as before containing 1.0% (v/v) Triton X—100 and incubated at 37°C for 45 mins. The suspension was then centrifuged at 100,000 g for 60 mins and the solubilised enzymes contained in the supernatant separated by DEAE-cellulose column chromatography. Enkephalinase A1 was further purified by Sephacryl S.300 chromatography.

Enkephalinase-inhibiting activity was estimated by the following procedure. 1.75 $\mu$l of leucine enkephalin (0.317 mg/ml), 0.5 $\mu$l of $^3$H-leucine enkephalin ([tyrosyl - 3,5 - $^3$H]Enkephalin(5 - L - Leucine), The Radiochemical Centre, Amersham, England) and 5.75 $\mu$l of buffer as before were incubated at 30°C for 10 mins with 2 $\mu$l of either a solution of test compound (in either 50% ethanol/0.1M sodium hydrogen carbonate or distilled water) or solvent alone as control. 10 $\mu$l of purified enkephalinase A1 at 30°C were added and incubation then continued for a further 30 mins (total incubation time 40 mins, final leucine enkephalin concentration $5 \times 10^{-5}$M, final $^3$H-leucine enkephalin concentration 12.5 $\mu$Ci/ml). At the completion of incubation 3 $\mu$l of 0.16 M hydrochloric acid was added and the incubation mixture cooled on ice.

Separation of (a) unchanged $^3$H-leucine enkephalin and (b) $^3$H-Tyr-Gly-Gly-OH ($^3$H—TGG), generated from (a) by enkephalinase A1 in the incubation mixture was effected by thin layer chromatography (plastic silica gel plates of 0.1 mm layer thickness, solvent system ethyl acetate: propan-2-ol: 5% (v/v) acetic acid, 2:2:1) using solutions of the cold compounds as carriers. After drying the materials were visualized with ninhydrin and appropriate areas of the plates cut out and placed in scintillation vials containing 50% methanol/0.1 M hydrochloric acid to elute the $^3$H label. Biofluor reagent (10 ml) was then added and the radioactivity determined by liquid scintillation counting.

The $^3$H—TGG generated in the presence of test compound (expressed as a percentage of the control figure) was then calculated and an approximate $IC_{50}$ figure (concentration of test compound required for 50% inhibition of $^3$H—TGG generation) then determined graphically.

B) The following method is a modification of the method of Malfroy *et al; Nature* Vol. 276, 30 November 1978 pages 523 to 526.

Three rats were killed by decapitation and the striata dissected out on ice. The pooled tissues were homogenised in ice-cold Tris/hydrochloric acid buffer (50 mM, pH 7.60, 10.0 ml) and centrifuged (1000 g, 10 mins). The resulting supernatant was recentrifuged (47,000 g, 20 mins) and the second supernatant discarded. The pellet was washed twice and finally resuspended in buffer as before (9.6 ml) and aliquots (1.9 ml) of this suspension incubated at 22°C. Five minutes after incubation commenced 20 $\mu$l of either a solution of the test compound (in either buffer as before or in ethanol or dimethylsulphoxide) or solvent alone as control was added followed after a further five minutes by a solution of $^3$H-leucine enkephalin (The Radiochemical Centre, Amersham, England) (80 $\mu$l, final concentration $3 \times 10^{-8}$M, 1.25 $\mu$Ci/ml) and incubation then continued for a further 30 mins. (total incubation time 40 mins). At the completion of incubation N hydrochloric acid (50 $\mu$l) was added, the mixture heated in a boiling water bath for 15 mins and precipitated material then removed by centrifugation (1500 g, 10 mins).

Separation of

(a) unchanged $^3$H-leucine enkephalin,

(b) $^3$H-Tyr-Gly-Gly-OH ($^3$H—TGG), generated from (a) by enkephalinase, and

(c) $^3$H-Tyr-OH, generated from both (a) and (b) by aminopeptidase(s)

in the supernatant and the subsequent procedure was carried out as described in method A) above. In this method, the $IC_{50}$ figure could be only approximate due to catabolism of $^3$H—TGG, once generated, by aminopeptidase(s) in the tissue homogenate (*vid.sup.).*

The results are set out in the following Table, the figures marked with an asterisk being obtained using method B, the others using method A. For comparison purpose, the $IC_{50}$ figure for captopril (obtained using method A above) is also given.

| Compound | Approx $IC_{50}$(M) |
|---|---|
| 1 | $1.7 \times 10^{-5}$ |
| 2 | $7.2 \times 10^{-6}$* |
| 3 | $4.0 \times 10^{-6}$* |
| 4 | $>1.0 \times 10^{-4}$ |
| 6 | $1.1 \times 10^{-5}$ |
| 7 | $1.4 \times 10^{-7}$ |
| 9 | $3.3 \times 10^{-7}$ |
| 10 | $>1.0 \times 10^{-4}$* |
| 11 | $>5 \times 10^{-4}$ |
| 12 | $1.9 \times 10^{-5}$ |
| 13 | $4.3 \times 10^{-6}$ |
| 14 | $6.5 \times 10^{-5}$ |
| 15 | $1.5 \times 10^{-7}$ |
| (Captopril | $3 \times 10^{-3}$) |

Antinociceptive activity

The antinociceptive activity of compound 1 was tested *in vivo* in mice using the following method:—

Method

A modification of the writhing assay of Siegmund *et al* (Proc. Soc. exp. Biol. Med., 1957, *95*: 727—731) was used. Compound 1 dissolved in saline, or saline alone, was administered intracerebroventricularly (i.c.v.) to groups of 5 mice at the doses shown below. 1 hour or 2 hours later, the mice were injected i.p. with the nonciceptive stimulus, i.e. 0.6% (v/v) aqueous acetic acid. 20 minutes after the injection, the number of writhes exhibited over a 2.5 minute period was counted. An antinociceptive compound will reduce the number of writhes and this may be expressed as a percentage inhibition:

$$\% \text{ inhibition} = \frac{\text{no of writhes (saline treated)} - \text{no of writhes (drug treated)} \times 100}{\text{no of writhes (saline treated)}}$$

Results

| Dose ($\mu$g mouse) | % inhibition of writhes Time prior to acetic acid | |
|---|---|---|
| | 1h | 2h |
| 40 | 75 | 84 |
| 20 | 32 | 44 |
| 10 | 28 | 48 |
| 5 | slight potentiation | 36 |

Pharmaceutical Formulations

The compound of formula (I) employed in the following Examples of pharmaceutical formulations may be any compound of formula (I) defined above or a basic salt thereof.

A) Tablet Formulation (0.5 mg/tablet)

| | |
|---|---|
| Compound of formula (I) | 0.5 mg |
| Maize Starch | 10 mg |
| Polyvinylpyrrolidone | 2 mg |
| Magnesium Stearate | 2 mg |
| Lactose | to 100 mg |

Mix together the compound of formula (I), Lactose and Maize Starch. Granulate with a solution of the Polyvinylpyrrolidone dissolved in water. Dry the granules, add the Magnesium Stearate and compress to produce tablets, 100 mg per tablet.

B) Suppository (0.5 mg/product)

| | |
|---|---|
| Compound of formula (I) | 25 mg |
| Suppository Base (Massa Esterinum C) | to 100 g |

Melt the suppository base at 40°C. Gradually incorporate the compound of formula (I) in fine powder form and mix until homogenous. Pour into suitable moulds, 2 g per mould, and allow to set.

Mass Esterinum is a commercially available suppository base consisting of a mixture of mono, di, and tri-glycerides of saturated vegetable fatty acids. It is marketed by Henkel International, Dusseldorf.

C) Pessary (0.5 mg/product)

| | |
|---|---|
| Compound of formula (I) | 0.5 mg |
| Lactose | 400 mg |
| Polyvinylpyrrolidone | 5 mg |
| Magnesium Stearate | 4.5 mg |

Mix together the compound of formula (I) and Lactose. Granulate with a solution of Polyvinylpyrrolidone in 50% aqueous ethanol. Dry the granules, add the Magnesium Stearate and compress on suitably shaped punches, 410 mg per pessary.

D) Freeze-dried Injection 0.5 mg/vial

| | |
|---|---|
| Compound of formula (I) | 0.5 mg |
| Mannitol | 99.5 mg |
| Water for Injections to | 2.0 ml |

Dissolve the compound of formula (I) and mannitol in the Water for Injections. Sterilise the solution by passage through a membrane filter, 0.2 μm pore size, collecting the filtrate in a sterile receiver. Fill into sterile glass vials, 2 ml/vial under aseptic conditions and freeze-dry. Close the vials with sterile rubber closures secured with an aluminium seal.

The injection is reconstituted prior to administration by the addition of a convenient volume of Water for Injections or sterile saline solution.

**Claims**

1. Compounds of the general formula

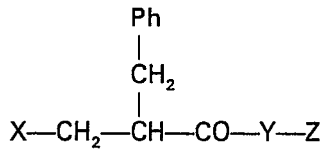

(I)

(wherein X is a group capable of functioning as a ligand for a zinc ion;

Ph is a phenyl group which is optionally substituted by one or more substituents selected from halo and nitro radicals;

Y is a group of formula:—

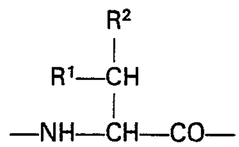

where

$R^1$ is hydrogen or methyl;

$R^2$ is alkyl of 1 to 3 carbon atoms or is methylthiomethyl; and

Z is a group of formula —$OR^3$ or —$NR^4R^5$ where $R^3$, $R^4$ and $R^5$ are each hydrogen or alkyl of 1 to 4 carbon atoms and $R^3$ can further be phenylalkyl having 1 to 3 carbon atoms in the alkylene moiety thereof, or phenyl) and pharmacologically acceptable basic salts thereof.

2. Compounds as claimed in claim 1 wherein X represents carboxyl.

3. Compounds as claimed in claim 1 wherein X represents mercapto or alkanoylthio having 2 to 5 carbon atoms.

4. Compounds as claimed in any of the preceding claims wherein Ph is unsubstituted phenyl.

5. Compounds as claimed in any of the preceding claims wherein Y is a group of formula

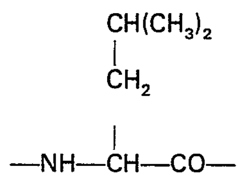

(in either the *D*- or *L*-configuration).

6. Compounds as claimed in any of claims 1 to 4 wherein Y is a group of formula:

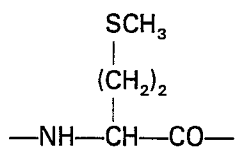

(in either the *D*- or *L*-configuration).

7. Compounds as claimed in any of the preceding claims wherein Z is a group of formula —OH or —$NH_2$.

8. A process for the preparation of a compound of formula I (as defined in claim 1) or a pharmacologically acceptable basic salt thereof which comprises reacting a compound of formula

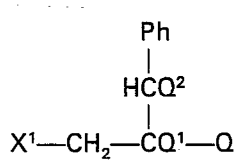

(II)

(wherein Ph is as defined in claim 1; $X^1$ is a group X (as defined in claim 1) or a functionally protected derivative thereof; Q is carboxyl or a functionally activated derivative thereof; and $Q^1$ and $Q^2$ are both hydrogen or together form a bond) with a compound of formula

$$H—Y—Z^1 \qquad (III)$$

(wherein Y is as defined in claim 1; and $Z^1$ is a group Z (as defined in claim 1) or a functionally protected derivative thereof); followed (when $Q^1$ and $Q^2$ together form a bond) by selective reduction of the said bond and, as appropriate, by deprotection of the product into the compound of formula (I) or pharmacologically acceptable basic salt thereof.

9. Pharmaceutical formulations comprising an amount of a compound of formula I (as defined in claim 1) or a pharmacologically acceptable basic salt thereof together with an acceptable carrier therefor.

10. Formulations as claimed in claim 9 adapted for oral, rectal, nasal, topical, vaginal or parenteral administration.

11. Formulations as claimed in claim 10 in the form of tablets, capsules, cachets or injectable solutions or suspensions.

12. Compounds of formula I (as defined in claim 1) and pharmacologically basic salts thereof, for use in the therapeutic treatment of a mammal.

13. Compounds of formula I (as defined in claim 1) and pharmacologically acceptable basic salts thereof, for use in the therapeutic treatment of a human.

14. Compounds of formula I (as defined in claim 1) and pharmacologically acceptable basic salts thereof, for use in the prolongation and/or potentiation in a mammal of the effects of endogenous or exogenous enkephalins.

15. Compounds of formula I (as defined in claim 1) and pharmacologically acceptable basic salts thereof, for use in the treatment of a mammal for a condition where an agent with a morphine-like effect is indicated.

## Revendications

1. Composés de la formule générale

```
              Ph
              |
              CH2
              |
X—CH2—CH—CO—Y—Z          (I)
```

(où X est un radical capable de fonctionner comme ligande pour un ion zinc;

Ph est un radical phényle qui est éventuellement substitué par un ou plusieurs substituants choisis entre halo et nitro;

Y est un radical de formule:

```
          R2
          |
     R1—CH
          |
    —NH—CH—CO—
```

où $R^1$ est un atome d'hydrogène ou un radical méthyle;

$R^2$ est un radical alcoyle de 1 à 3 atomes de carbone ou est un radical méthylthiométhyle, et

Z est un radical $—OR^3$ ou $—NR^4R^5$, où $R^3$, $R^4$ et $R^5$ sont chacun un atome d'hydrogène ou un radical alcoyle de 1 à 4 atomes de carbone et $R^3$ peut être en outre un radical phénylalcoyle comptant 1 à 3 atomes de carbone dans sa partie alcoylène ou un radical phényle) et leurs sels basiques pharmacologiquement acceptables.

2. Composés suivant la revendication 1, dans la formule desquels X est un radical carboxyle.

3. Composés suivant la revendication 1, dans la formule desquels X est un radical mercapto ou alcanoylthio de 2 à 5 atomes de carbone.

4. Composés suivant l'une quelconque des revendications précédentes, dans la formule desquels Ph est un radical phényle non substitué.

5. Composés suivant l'une quelconque des revendications précédentes, dans la formule desquels Y est un radical de formule:

```
          CH(CH3)2
          |
          CH2
          |
    —NH—CH—CO—
```

(ayant la configuration *D* ou *L*).

6. Composés suivant l'une quelconque des revendications 1 à 4, dans la formule desquels Y est un radical de formule:

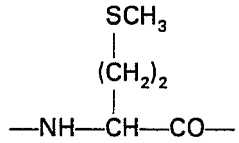

(ayant la configuration *D* ou *L*).

7. Composés suivant l'une quelconque des revendications précédentes, dans la formule desquels Z est un radical de formule —OH ou —$NH_2$.

8. Procédé de préparation d'un composé de formule I (tel que défini dans la revendication 1) ou d'un sel basique pharmacologiquement acceptable de celui-ci, qui comprend la réaction d'un composé de formule

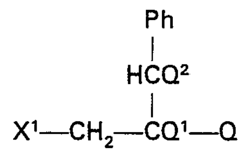

(II)

(où Ph est tel que défini dans la revendication 1, $X^1$ est un radical X (tel que défini dans la revendication 1) ou un dérivé fonctionnellement protégé de celui-ci; Q est un radical carboxyle ou un dérivé fonctionnellement protégé de celui-ci et $Q^1$ et $Q^2$ sont tous deux des atomes d'hydrogène ou forment ensemble une liaison)
avec un composé de formule

$$H—Y—Z^1 \qquad (III)$$

(où Y est tel que défini dans la revendication 1; et $Z^1$ est un radical Z (tel que défini dans la revendication 1) ou un dérivé fonctionnellement protégé de celui-ci)
puis (lorsque $Q^1$ et $Q^2$ forment ensemble une liaison) la réduction sélective de cette liaison et, suivant le cas, la déprotection du produit en le composé de formule (I) ou un sel basique pharmacologiquement acceptable de celui-ci.

9. Formulations pharmaceutiques comprenant une quantité d'un composé de formule I (tel que défini dans la revendication 1) ou d'un sel basique pharmacologiquement acceptable de celui-ci, outre un excipient acceptable.

10. Formulations suivant la revendication 9 se prêtant à l'administration par voie orale, rectale, nasale, topique, vaginale ou parentérale.

11. Formulations suivant la revendication 10, sous la forme de comprimés, de capsules, de cachets ou de solutions ou suspensions à injecter.

12. Composés de formule I (tels que définis dans la revendication 1) et leurs sels basiques pharmacologiquement acceptables à utiliser dans le traitement thérapeutique d'un mammifère.

13. Composés de formule I (tels que définis dans la revendication 1) et leurs sels basiques pharmacologiquement acceptables à utiliser dans le traitement thérapeutique de l'être humain.

14. Composés de formule I (tels que définis dans la revendication 1) et leurs sels basiques pharmacologiquement acceptables à utiliser dans la prolongation et/ou la potentialisation chez un mammifère des effets des enképhalines endogènes ou exogènes.

15. Composés de formule I (tels que définis dans la revendication 1) et leurs sels basiques pharmacologiquement acceptables à utiliser dans le traitement d'un mammifère pour une affection où un agent ayant un effet morphinomimétique est indiqué.

**Patentansprüche**

1. Verbindungen der allgemeinen Formel I

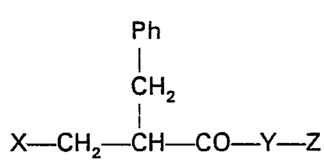

(I)

(worin X eine Gruppe ist, die als ein Ligand für ein Zinkatom dienen kann;

Ph einen gegebenenfalls durch einen oder mehrere Substituenten, die unter Halogenatomen und

Nitrogruppen ausgewählt sind, substituierten Phenylrest darstellt,

Y einen Rest der Formel

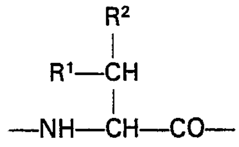

bedeutet worin

$R^1$ ein Wasserstoffatom oder eine Methylgruppe darstellt,

$R^2$ einen Alkylrest mit 1 bis 3 Kohlenstoffatomen oder Methylthiomethyl bedeutet, und

Z eine Gruppe der Formel —$OR^3$ oder —$NR^4R^5$ ist, worin $R^3$, $R^4$ und $R^5$ jeweils ein Wasserstoffatom oder einen Alkylrest mit 1 bis 4 Kohlenstoffatomen darstellen und $R^3$ ferner ein Phenylalkylrest mit 1 bis 3 Kohlenstoffatomen in dem Alkylenteil davon sein kann, oder Phenyl) und pharmakologisch verträgliche basische Salze davon.

2. Verbindungen nach Anspruch 1, worin X einen Carboxylrest darstellt.

3. Verbindungen nach Anspruch 1, worin X einen Mercapto- oder Alkanoylthiorest mit 2 bis 5 Kohlenstoffatomen bedeutet.

4. Verbindungen nach einem der voranstehenden Ansprüche, worin pH einen unsubstituierten Phenylrest darstellt.

5. Verbindungen nach einem der voranstehenden Ansprüche, worin Y einen Rest der Formel

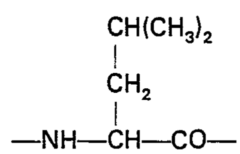

(entweder in der D- oder L-Konfiguration) bedeutet.

6. Verbindungen nach einem der Ansprüche 1 bis 4, worin Y einen Rest der Formel

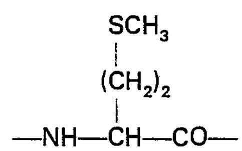

(entweder in der D- oder der L-Konfiguration) bedeutet.

7. Verbindungen nach einem der voranstehenden Ansprüche, worin Z ein Rest der Formel —OH oder —$NH_2$ ist.

8. Verfahren zur Herstellung einer Verbindung nach Formel I (gemäß der Definition in Anspruch 1) oder eines pharmakologisch verträglichen basischen Salzes davon, wobei eine Verbindung der allgemeinen Formel II

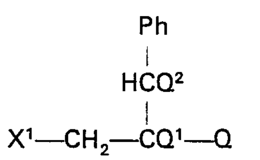

(II)

(worin Ph die in Anspruch 1 angegebene Bedeutung hat; $X^1$ einen Rest X (gemäß der Definition in Anspruch 1) oder ein funktional geschütztes Derivat davon bedeutet; Q eine Carboxylgruppe oder ein funktional aktiviertes Derivat davon bedeutet; und $Q^1$ und $Q^2$ beide ein Wasserstoffatom darstellen oder zusammen eine Bindung bilden) mit einer Verbindung der allgemeinen Formel III

H—Y—$Z^1$ (III)

umgesetzt wird (worin Y die in Anspruch 1 angegebene Bedeutung hat, und $Z^1$ einen Rest Z (gemäß der Definition in Anspruch 1) oder ein funktional geschütztes Derivat davon bedeutet), woran sich (wenn $Q^1$ und $Q^2$ zusammen eine Bindung bilden) eine selektive Reduktion der Bindung anschließt und gegebenenfalls eine Schutzgruppenabspaltung bei dem Produkt zu der Verbindung nach Formel I oder einem pharmakologisch verträglichen basischen Salz davon.

9. Pharmazeutische Formulierungen, die eine Menge einer Verbindung nach Formel I (gemäß

der Definition in Anspruch 1) oder eines pharmakologisch verträglichen basischen Salzes davon zusammen mit einem verträglichen Träger dafür enthalten.

10. Formulierungen nach Anspruch 9, die zur oralen, rektalen, nasalen, äußerlichen, vaginalen oder parenteralen Verabfolgung angepaßt sind.

11. Formulierungen nach Anspruch 10 in Form von Tabletten, Kapseln, Cachets oder injizierbaren Lösungen oder Suspensionen.

12. Verbindungen nach Formel I (gemäß der Definition in Anspruch 1) und pharmakologisch verträgliche basische Salze davon zur Verwendung in der therapeutischen Behandlung eines Säugetiers.

13. Verbindungen nach Formel I (gemäß der Definition in Anspruch 1) und pharmakologisch verträgliche basische Salze davon zur Verwendung in der therapeutischen Behandlung eines Menschen.

14. Verbindungen nach Formel I (gemäß der Definition in Anspruch 1) und pharmakologisch verträgliche basische Salze davon zur Verwendung für die Verlängerung und/oder Potenzierung der Wirkungen von endogenen oder exogenen Encephalinen in einem Säugetier.

15. Verbindungen nach Formel I (gemäß der Definition in Anspruch 1) und deren pharmakologisch verträgliche basische Salze zur Verwendung in der Behandlung eines Säugetiers bei einem Zustand, bei dem ein Mittel mit einem Morphin-ähnlichen Effekt indiziert ist.